# EUROPÄISCHE PATENTSCHRIFT

(11) **EP 0 623 586 B1**
(45) Veröffentlichungstag und Bekanntmachung des Hinweises auf die Patenterteilung: **03.04.1996**
(21) Anmeldenummer: 94106841.3
(22) Anmeldetag: 02.05.1994
(51) Int. Cl.: C07C 209/72, C07C 211/25

(54) **Verfahren zur Herstellung von 2-(1-Cyclohexenyl)ethylamin**
Process for the preparation of 2-(1-cyclohexenyl)ethylamine
Procédé pour la préparation 2-(1-cyclohexényl)éthylamine

(30) Priorität: 03.05.1993 CH 1349/93
(43) Veröffentlichungstag der Anmeldung: 09.11.1994
(73) Patentinhaber: LONZA AG, CH-3945 Gampel/Wallis (CH)
(72) Erfinder: Kuo, David L., Dr., Brig (Kanton Wallis) (CH); Eyer, Martin, Dr., Glis (Kanton Wallis) (CH)
(74) Vertreter: Weinhold, Peter, Dr.

(56) Entgegenhaltungen:
- JOURNAL OF THE AMERICAN CHEMICAL SOCIETY., Bd.77, Nr.22, 26. November 1955, GASTON, PA US Seiten 6042 - 6045 R. A. BENKESER ET AL. 'Reduction of organic compounds by lithium in low molecular weight amines.'
- JOURNAL OF THE AMERICAN CHEMICAL SOCIETY., Bd.80, Nr.24, 8. Januar 1959, GASTON, PA US Seiten 6573 - 6577 ROBERT A. BENKESER ET AL. 'Reduction of organic compounds by lithium in low molecular weight amines.'

## Beschreibung

Die Erfindung beinhaltet ein neues Verfahren zur Herstellung von 2-(1-Cyclohexenyl)ethylamin der Formel 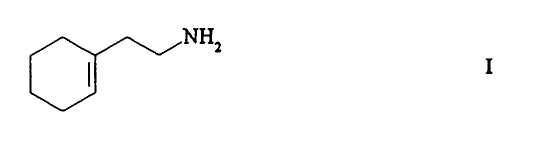
2-(1-Cyclohexenyl)ethylamin ist u. a. ein Schlüsselzwischenprodukt für die Herstellung des hustenreizmildernden Pharmazeutikums Dextromethorphan. (EP-A 315 886).

Bekannt ist, 2-(1-Cyclohexenyl)ethylamin durch Reduktion von Benzylcyanid mit Lithium in Gegenwart von Ethylamin herzustellen (R.A. Benkeser et. al J. Am. Chem. Soc. 1955, 77 6042). Aus J. Am. Chem. Soc. 1959, 80, 6573 (R.A. Benkeser et al.) ist die Herstellung von N-Phenyl substituiertem 2-(1-Cyclohexenyl)ethylamin durch Reduktion von N-Phenyl, 2-Phenylethylamin mit Lithium in Gegenwart von Methylamin bekannt.

Das gewünschte Produkt wird dabei in einer Ausbeute von ca. 50%, zusammen mit ca 6% Anteil des vollständig reduzierten Cyclohexylethylamins als Nebenprodukt erhalten. Neben der bescheidenen Ausbeute ist der beträchtliche Anteil des sehr schwer destillativ abtrennbaren Nebenproduktes von grossem Nachteil.

Im Hinblick auf eine Synthese von 2-(1-Cyclohexenyl)ethylamin im technischen Masstab bestand daher die Aufgabe ein Verfahren auszuarbeiten, dass die genannten Nachteile nicht aufweist.

Gelöst werden konnte die Aufgabe mit dem neuen Verfahren gemäss Patentanspruch 1.

Ausgangsprodukt dieses Verfahrens ist das grosstechnisch erhältliche Phenylethylamin, welches erfindungsgemäss mit Lithium in Gegenwart eines primären oder sekundären aliphatischen Alkylamins reduziert wird.

Zweckmässig wird so vorgegangen, dass das 2-Phenylethylamin in einem Überschuss von primärem oder sekundären aliphatischen Alkylamin vorgelegt wird.
Dabei dient das primäre oder sekundäre aliphatische Alkylamin gleichzeitig als Lösungsmittel. Die Zugabe eines zusätzlichen inerten Lösungsmittels ist grundsätzlich möglich, erübrigt sich aber in der Regel.
Zweckmässig werden die primären oder sekundären aliphatischen (C₁-C₄)-Alkylamine, bevorzugt die primären aliphatischen (C₁-C₄)-Alkylamine wie Methylamin, Ethylamin, n- oder i-Propylamin oder n-, i- oder t-Butylamin eingesetzt. Besonders bevorzugt ist Ethylamin.

Lithium wird bevorzugt in Pulverform dem Eduktgemisch zugegeben.
Bezogen auf 1 mol 2-Phenylethylamin werden 3.8 mol bis 4.2 mol Lithium eingesetzt.
Die Reduktion erfolgt bei einer Temperatur zwischen -100°C und -10°C.

Zweckmässig erfolgt die Umsetzung in einer Inertgasatmosphäre.

Nach einer Umsetzungszeit von ca. 6 h bis 14 h kann das 2-(1-Cyclohexenyl)ethylamin auf fachmännische Weise isoliert werden. Zweckmässig wird zunächst destillative der Überschuss an primärem oder sekundären aliphatischem Alkylamin abgetrennt und darauf das Produkt mit einem geeigneten Lösungsmittel aus dem Reaktionsgemisch extrahiert.

Der Gehalt an vollständig reduziertem Cyclohexylethylamin im 2-(1-Cyclohexenyl)ethylamin kann mit dem erfindungsgemässen Verfahren gegenüber dem genannten Verfahren aus dem Stand der Technik auf einen Drittel, d. h. auf ca. 2%, gesenkt werden.

### Beispiel

125 ml Ethylamin wurden in einem 250 ml Dreihalskolben, der zuvor mit Argon gespült wurde, bei -70°C vorgelegt. Daraufwurden 6.27 g (50 mmol) 2-Phenylethylamin zugegeben. Der resultierenden, klaren, farblosen Lösung wurden dann 1.14g (164.2 mmol) Li-Pulver zudosiert, worauf nach einer einsetzenden Trübung die Lösung nach 5 min eine dunkelblaue Farbe annahm. Nach 2 Stunden rühren bei -70°C wurde auf -30°C erwärmt. Dann wurden nochmals 0.24g (34.6 mmol) Li-Pulver zugesetzt. Nach erneutem 2stündigem Rühren bei - 30°C wurde das Kühlbad entfernt. Das Reaktionsgemisch liess man über Nacht auf Raumtemperatur erwärmen.
Dem Reaktionsgemisch gab man dann 25 ml Methanol abs. zu, worauf sich eine graue Suspension bildete. Nach weiteren 20 Minuten rühren bei Raumtemperatur gab man langsam 25 ml Wasser zu (Exothermie 40°C). Darauf wurde bei 40°C der Ethylaminüberschuss abdestilliert.
Der Destillationsrückstand wurde zur Trockene eingedampft. Dann wurden 100 ml Wasser zugegeben und 3mal mit 80 ml Chloroform extrahiert.
Die vereinigten organischen Phasen wurden über Na₂SO₄ getrocknet, filtriert und eingedampft.
Man erhielt das 2-(1-Cyclohexenyl)ethylamin in einer Ausbeute gemäss GC von 69.8%. Der Gehalt an Cyclohexylethylamin betrug gemäss GC 2%.

## Patentansprüche

1. Verfahren zur Herstellung von 2-(1-Cyclohexenyl)ethylamin, dadurch gekennzeichnet, daß 2-Phenylethylamin mit Lithium in Gegenwart eines primären oder sekundären aliphatischen Alkylamins bei einer Temperatur zwischen -100°C und -10°C reduziert wird, wobei bezogen auf 1 Mol 2-Phenylethylamin 3,8 Mol bis 4,2 Mol Lithium verwendet wird.

2. Verfahren nach Patentanspruch 1, dadurch gekennzeichnet, daß die Reduktion mit einem primären aliphatischen (C₁-C₄)-Alkylamin durchgeführt wird.

3. Verfahren nach Patentanspruch 1 oder 2, dadurch gekennzeichnet, daß die Reduktion mit Ethylamin durchgeführt wird.

## Claims

1. A process for the preparation of 2-(1-cyclohexenyl)-ethylamine, characterized in that 2-phenylethylamine is reduced with lithium in the presence of a primary or secondary aliphatic alkylamine at a temperature between -100°C and -10°C, wherein 3.8 to 4.2 moles of lithium are used relative to 1 mole of 2-phenylethylamine.

2. The process of claim 1, characterized in that the reduction is carried out with a primary aliphatic (C₁-C₄)-alkylamine.

3. The process of claim 1 or 2, characterized in that the reduction is carried out with ethylamine.

## Revendications

1. Procédé pour la préparation de la 2-(1-cyclohexényl)éthylamine, caractérisé en ce que l'on réduit la 2-phényléthylamine avec du lithium en présence d'une alkylamine aliphatique primaire ou secondaire à une température entre -100°C et -10°C, après quoi on utilise 3,8 moles jusqu'à 4,2 moles de lithium rapportés à 1 mole de 2-phényléthylamine.

2. Procédé selon la revendication 1, caractérisé en ce que la réduction est conduite avec une alkylamine (C₁-C₄) aliphatique primaire.

3. Procédé selon la revendication 1 ou 2, caractérisé en ce que la réduction est conduite avec de l'éthylamine.
